# EUROPEAN PATENT SPECIFICATION

(11) **EP 4 499 022 B1**
(45) Date of publication and mention of the grant of the patent: **10.12.2025**
(21) Application number: 23712285.8
(22) Date of filing: 21.03.2023
(51) Int. Cl.: A61K 8/02, A61K 8/19, A61K 8/25, A61K 8/26, A61K 8/37, A61K 8/73, A61K 8/92, A61Q 15/00, A61K 8/9789

(54) **COMPOSITION WITH ENHANCED STABILITY AND SENSORY**
ZUSAMMENSETZUNG MIT VERBESSERTER STABILITÄT UND SENSORIK
COMPOSITION PRÉSENTANT STABILITÉ ET SENSORIALITÉ AMÉLIORÉES

(30) Priority: 28.03.2022 EP 22164763
(43) Date of publication of application: 05.02.2025
(73) Proprietor: Unilever IP Holdings B.V., 6708 WH Wageningen (NL); Unilever Global IP Limited, Wirral, Merseyside CH62 4ZD (GB)
(72) Inventor: ACUNA, Alyssa Grace, 6708 WH Wageningen (NL); MELO, Kathleen Lynn, 6708 WH Wageningen (NL); METRAUX, Gabriella Serena, 6708 WH Wageningen (NL)
(74) Representative: Turner, Felicity Margaret Mary
(86) International application number: PCT/EP2023/057175
(87) International publication number: WO 2023/186618

(56) References cited:
- US-A- 4 388 301
- US-A1- 2020 078 275
- US-A1- 2020 383 886
- US-B1- 10 758 476
- DATABASE GNPD [online] MINTEL; 23 March 2020 (2020-03-23), ANONYMOUS: "Keep Cool Deodorant Stick", XP055915719, retrieved from https://www.gnpd.com/sinatra/recordpage/7456923/ Database accession no. 7456923

## Description

### Field of the Invention

Disclosed herein is a personal care composition. In particular, disclosed herein is an anhydrous personal care composition comprising a first mineral compound that is a clay mineral; a second mineral compound that is an oxide mineral; an inorganic salt; a powdered hygroscopic material; and a plant-based oil, wax, or mixture thereof. The disclosed personal care composition is especially useful as an efficacious, natural deodorant alternative that possess enhanced stability, is free of astringent metal salts, and provides a pleasant consumer use experience upon topical application.

### Background of the Invention

Perspiration, an innate means of thermoregulation in humans, is the production of sweat, which is a fluid secretion containing a mixture of protein, lipid, and other organic compounds. This fluid is initially odorless until bacteria on the skin metabolize and decompose the sweat, resulting in malodorous sweat by-products.

Personal care compositions, such as antiperspirants and deodorants, used to provide a malodor protection benefit upon topical application to desired surfaces of the human body, namely the underarm regions, viz., the axilla, often comprise an astringent metal salt as antiperspirant active, notably aluminum or zirconium salt. While effective as antiperspirant actives, such salts may be perceived as harsh or irritating to the skin and/or the cause of fabric discoloration in clothing by some consumers and there is thus a need for alternative means of achieving fresh underarms. By extension, there is also an increasing consumer interest in natural deodorant options to address common concerns such as unpleasant use experience upon topical application to such body parts without compromising performance of such products compared to their conventional antiperspirant counterparts.

Deodorants which are free of astringent metal salts are already available commercially, for example, Native's (e.g., Native^{™} Unscented Deodorant). However, the compositions of many commercially available natural deodorants may provide for sticky, wet, or other unpleasant sensory experiences, and/or still cause irritation to the consumer.

The composition disclosed herein is particularly concerned with anhydrous personal care compositions comprising a first mineral compound that is a clay mineral; a second mineral compound that is an oxide mineral; an inorganic salt; a powdered hygroscopic material; and a plant-based oil, wax, or mixture thereof; wherein the composition unexpectedly exhibits enhanced stability. The product of the invention is related to a formulation dispensed in the form of a solid.

### Additional Information

Efforts have been disclosed for making anhydrous personal care compositions comprising clay mineral and inorganic salt.

U.S. Patent No. 3,819,671 and 3,929,986 relate to antiperspirant compositions comprising a dioxaluminin or derivative thereof as antiperspirant active, wherein clay and magnesium carbonate are disclosed as conventional powders for use in a base.

U.S. Patent No. 5,165,915 discloses a spherical clay mineral powder or spherical composite comprising water-swellable clay mineral that can be formulated for use in cosmetics and deodorants.

Citation from database GNPD (MINTEL) with accession no. 7456923 discloses a deodorant Stick comprising magnesium hydroxide, sunflower seed oil, sodium bicarbonate, Maranta arundinacea (arrowroot) root powder, Copernicia cerifera (carnauba) wax, Butyrospermum parkii (shea) butter, kaolin (white clay), magnesium carbonate hydroxide and argilla (yellow clay).

None of the additional information describes a stable, anhydrous personal care composition as described herein, and particularly, one comprising a first mineral compound that is a clay mineral; a second mineral compound that is an oxide mineral; an inorganic salt; a powdered hygroscopic material; and a plant-based oil, wax, or mixture thereof.

### Summary of the Invention

There is a common problem observed with natural stick formulations wherein such formulations are not stable due to oil and wax structure separation or structurant systems that are not sufficiently robust to support solid formulations that are high powder suspensions.

In a first aspect, there is provided a personal care composition comprising a first mineral compound that is a clay mineral; a second mineral compound that is an oxide mineral; an inorganic salt; powdered hygroscopic material; and plant-based oil, wax, or mixture thereof, wherein the first mineral compound to second mineral compound ratio is 1:3 to 1:2, preferably, 1:3 to 1:2.5.

In a second aspect, the personal care composition disclosed is natural, naturally derived, or a combination thereof.

In a third aspect, the disclosed personal care composition provides for a solid product comprising the composition of the first aspect comprised within appropriate packaging formats, including a stick dispenser product package.

It is preferable that the composition is a solid product comprised within a stick dispenser product package.

In a fourth aspect, there is provided a method of attaining deodorizing benefits comprising the steps of: (a) identifying skin in need of deodorization, preferably, the axillae; and (b) topically applying, directly or indirectly, to the skin a composition made according to the first aspect of the compositions disclosed herein.

In a fifth aspect, there is provided a use of a personal care composition according to the first aspect for delivering a deodorancy benefit.

In a sixth aspect, there is provided a method of manufacturing the personal care composition according to the first aspect.

All other aspects of the disclosed personal care composition will become more readily apparent upon considering the detailed description, data, and examples which follow.

### Detailed Description of the Invention

For the avoidance of doubt, the term "comprising" is meant not to be limiting to any stated elements but rather to encompass non-specified elements of major or minor functional importance. Therefore, the listed steps, elements or options need not be exhaustive. Whenever the words "including" or "having" are used, these terms are meant to be equivalent to "comprising" as defined above. All ranges are meant to include all those ranges subsumed therein.

Herein, any feature of one aspect of the disclosed composition may be utilized in any other aspect of the composition. Any feature described as 'preferred' should be understood to be particularly preferred in combination with a further preferred feature or features.

Herein, when a component is referred to in the singular, it is to be understood that multiple components of the type referred to could be present and all should be included in calculating any amount or ratio specified.

The term "skin" as used herein includes the skin on the face, neck, chest, back, arms, axilla, buttocks, hands, legs and scalp. Skin benefit agent, as used herein, is meant to include a component that (a) improves a facial or body characteristic after topical application like a skin characteristic, (b) benefits the same, or (c) both (a) and (b). The personal care composition, as described herein, may be an end use composition. The end use composition is a leave-on composition. In another aspect, the end use composition is a deodorant. The term "leave-on" as used with reference to compositions herein means a composition that is applied to or rubbed on the skin, and left thereon. The end use compositions of the present have been developed for applications that include non-therapeutic and cosmetic topical applications to skin.

Substantially free of, as used herein, is intended to mean comprising less than 2%, preferably, less than 1% by weight, more preferably, less than 0.5% by weight, and, most preferably, 0% by weight. Anhydrous, as used herein, is intended to mean comprising 0% by weight of any added water. "Free from aluminum and/or zirconium salt" or "in absence of aluminum and/or zirconium salt" refers to a composition containing less than 1%, preferably less than 0.1% and more preferably, containing 0% of added aluminum and/or zirconium salt. The first and second compounds of the personal care composition disclosed herein are different materials, preferably, the second material is not a clay mineral. As used herein, dioctahedral orientation refers to having two of three available octahedrally coordinated positions occupied, while trioctahedral orientation refers to having all three available octahedrally coordinated positions occupied. "Hygroscopic material," as used herein, refers to a material that absorbs moisture from the air or its environment. The term "suspension" as used with reference herein means a mixture in which particles are dispersed in the bulk of a fluid. As used herein, a high powder composition means that the composition comprises greater than or equal to 40% total powdered content. The personal care compositions disclosed herein are high powder suspensions, that is, solid structures formed from a high powdered suspension. As used herein, "natural" or "naturally derived" refers to the composition being free of artificial ingredients. Specifically, a "naturally derived" ingredient or component means that its starting material originates from nature (e.g., plants or minerals), but may have subsequently been processed by man to render the material desirable for use.

All states of matter, as used herein, such as solid, liquid and gas relate to the specified state of matter at 25°C and atmospheric pressure. Herein, references to densities should be understood to be densities measured at 25°C and atmospheric pressure using standard methods. References to the stability of deodorant sticks should be understood to be assessments of color, odor, and appearance of the sticks at 45°C for 12 weeks.

Except in the examples, or where otherwise explicitly indicated, all numbers in this description indicating amounts of material are to be understood as modified by the word "about." All amounts are by total weight of the composition, unless otherwise specified. Use of "average" herein refers to the number average.

Personal care compositions as disclosed herein comprise a first mineral compound that is a clay mineral. The term "clay" commonly refers to a number of fine-sized particles in sediment, soil, or rock, and its individual particles often possess diameters of less than 5 micrometers (µm). Most clays are composed of clay minerals, such as antigorite, attapulgite, glauconite, kaolinite, montmorillonite and zeolite. It is within the scope of the disclosed personal care composition to employ clay mineral that is natural and pure, preferably, minimally processed and/or modified during its extraction process. It is preferred that the clay mineral is a crystalline silicate.

Clays, namely silicate clays, are classified into different groups based on the number and arrangement of tetrahedral (e.g., silica) and octahedral (e.g., alumina-magnesia) sheets contained in the crystal units or layers: (a) 1:1 type clay minerals; (b) 2:1 type clay minerals; and (c) 2:1:1 (or) 2:2 type clay minerals. The basic building blocks of tetrahedral and octahedral sheets are the silica tetrahedron and the aluminum octahedron. Clay minerals desirable for use in the disclosed personal care composition includes an asymmetric 1:1 or 2:1:1 clay particle having alternating tetrahedral and an octahedral sheet terminating with a tetrahedral and an octahedral sheet at exterior surface planes. In an aspect of the personal care composition, 1:1 and 2:1:1 clay may be combined.

The octahedral sheet preferably has coordinating octahedral cation of aluminum. The octahedral sheet may also comprise isomorphously substituted coordinating octahedral cations which are not aluminum. Isomorphously substituted coordinating octahedral cations include cations of magnesium or iron. It is preferred that the first mineral compound possesses an average particle size ranging from 0.001 to 60 microns, preferably, 0.01 to 50 microns, more preferably, 0.05 to 40 microns. In a particularly preferred aspect, the average particle size ranges from 0.1 to 10 microns.

Particles of 1:1 clay is particularly preferred as the clay mineral. Preferred 1:1 clays include kaolinite and serpentine subgroups of minerals. The species included within the kaolinite subgroup include but are not limited to kaolinite, dickite, halloysite, and nacrite. The species within the serpentine subgroup include but are not limited to chrysolite, lizardite, and amesite. Where the clay mineral is a 1:1 clay mineral, it is preferred that the mineral structure is characterized by a dioctahedral orientation. The octahedra preferably comprises a trivalent cation such as aluminum (e.g., Al³⁺) or iron (e.g., Fe³⁺), which results in two out of three available octahedrally coordinated positions being occupied.

In a particularly preferred aspect, the clay mineral is kaolin. The kaolin in compositions disclosed herein is a colloidal (e.g., particles from 1 to 1000 nanometers (nm)) clay mineral having a chemical composition comprising Al₂Si₂O₅(OH)₄. The colloidal kaolin will comprise 40 to 50% by weight SiO₂, 35 to 45% by weight Al₂O₃ and 1 to 2.5% by weight TiO₂. Kaolin may be used alone as a clay mineral according to the disclosed composition or may be used in conjunction with other clay minerals desirable for use in personal care products. It is desirable to use kaolin commercially made available by suppliers such as Jarchem Industries, Inc. under the trade name Jarxotic^{™} WC-K. In one aspect, the amount of kaolin in the personal care composition is 0.1 to 25%, preferably, 0.5 to 20%, and, most preferably, 1 to 15% by weight of the composition. Even more preferred, personal care compositions as disclosed herein comprise clay mineral from 2 to 12% by weight of the personal care composition.

Preferred 2:1:1 clays include chlorite group of minerals. The chlorite comprises tetrahedral-octahedral tetrahedral sheets like 2:1 clays, with an extra weakly bound brucite like layer between tetrahedral layers. The tetrahedral sheet preferably comprises coordinating tetrahedral cations of silicon. The tetrahedral sheet may also include isomorphously substituted coordinating tetrahedral cations which are not silicon. Isomorphously substituted coordinating tetrahedral cations include, but are not limited to, cations of aluminum, iron, or boron.

In an aspect of the disclosed personal care composition, bentonite is desirable for use as a 2:1:1 clay. The bentonite desirable for use in the personal care composition is made commercially available from suppliers like H&H Clay, Inc., Wyoming Bentonite, Brenntag Specialties, Inc. and Sigma Aldrich. Bentonite makes up from 0.01 to 10% by weight of the personal care composition. In another aspect, bentonite makes up from 0.1 to 8% by weight of the personal care composition, and in still another aspect, the personal care composition comprises 0.5 to 5% by weight bentonite, based on total weight of the personal care composition. In still another aspect, bentonite may be used in combination with kaolin.

Personal care compositions as disclosed herein comprise a second mineral compound that is an oxide mineral. Where the mineral is an oxide mineral, the octahedra preferably contains a divalent cation such as magnesium (e.g., Mg²⁺), calcium (e.g., Ca²⁺), or iron (e.g., Fe²⁺), which allows for a balanced overall charge when all three octahedral sites are occupied. In a particularly preferred aspect, the oxide mineral is a trioctahedral mineral, including but not limited to brucite (Mg(OH)₂) and portlandite (Ca(OH)₂). The oxide mineral desirable for use comprises Mg(OH)₂ (magnesium hydroxide) or Ca(OH)₂ (calcium hydroxide) or Mg(OH)₂ (magnesium hydroxide) and Ca(OH)₂ (calcium hydroxide). Preferably the oxide mineral is Mg(OH)₂ (magnesium hydroxide). Oxide mineral is a trioctahedral mineral means that the structure of the oxide mineral is trioctahedral. It is preferred that second mineral compound possesses average particle size ranging from 0.001 to 60 microns, preferably, 0.01 to 50 microns, more preferably, 0.05 to 40 microns. In a particularly preferred aspect, the average particle size ranges from 0.1 to 10 microns. The personal care composition comprises from 1 to 50%, preferably, 2 to 40%, more preferably, 3 to 35% of the second mineral compound by weight of the composition. In another aspect, the personal care composition comprises from 8 to 35% of the second mineral compound by weight of the composition.

In one aspect, the weight ratio of the first mineral compound to second mineral compound is 1:3 to 1:2, preferably, 1:3 to 1:2.5, and, more preferably, 1:3. In another aspect, the personal care composition comprises kaolin and magnesium hydroxide. In yet another aspect, the weight ratio of kaolin to magnesium hydroxide is 1:3 to 1:2, preferably, 1:3 to 1:2.5, and, more preferably, 1:3.

In yet another aspect, the personal care composition comprises a total mineral compound content (e.g., first and second mineral compounds) at least 10% by weight of the composition, preferably, at least 30%, preferably, 35 to 60%, more preferably, 40 to 50% by weight of the composition.

In still another aspect, the personal care compositions may further comprise other optional classes of minerals, preferably other crystalline silicate. Other natural clays such as smectite, sulphur, halite, sylvite, kieserite, talc, titanium dioxide, zinc oxide, iron oxide, black clay, blue clay, green clay, nude clay, pink clay, red clay, white clay and yellow clay, all of which may be in their pure forms or a blend of the aforementioned clay minerals, optionally comprising quartz, diatomaceous earth, or silica.

Personal care compositions as disclosed herein comprise at least one inorganic salt. Inorganic salts desirable for use in the disclosed personal care composition include metal carbonates, preferably, alkali and alkaline earth metal carbonates such as calcium carbonate, magnesium carbonate, sodium carbonate, and sodium bicarbonate. It is preferred that the inorganic salt is magnesium carbonate or calcium carbonate, more preferably, magnesium carbonate. Inorganic salts are incorporated in the composition at 0.01 to 15%, preferably, 0.1 to 12%, more preferably, 0.25 to 7%, and, most preferably, 0.5 to 5% by weight of the composition.

The combination of the first mineral compound; the second mineral compound; inorganic salt; powdered hygroscopic material; and plant-based oil, wax, or mixture thereof according to the personal care compositions disclosed herein unexpectedly provides for a personal care composition that is stable at elevated temperatures (e.g., 45°C for 12 weeks).

It is within the scope of the disclosed personal care composition to comprise a powdered hygroscopic material. The powdered hygroscopic material may be incorporated in the disclosed personal care composition in amounts ranging from 0.5 to 42%, from 0.5 to 41.8 wt%, from 0.5 to 40%, preferably, from 1 to 38% by weight of the composition, and, more preferably, from 2 to 37% powdered hygroscopic material by weight of the composition. In another aspect, it is provided that the disclosed personal care composition comprises 3 to 15%, preferably, 5 to 12% powdered hygroscopic material by weight of the composition. Desirable powdered hygroscopic materials for use in the disclosed personal care compositions include diatomaceous earth, silica, plant-based starches, non-modified cellulose (e.g., cellulose microfibrils, cellulose nanocrystals, or microcrystalline cellulose), and mixtures thereof. Preferably the powdered hygroscopic materials for use in the disclosed personal care compositions include plant-based starches, non-modified cellulose (e.g., cellulose microfibrils, cellulose nanocrystals, or microcrystalline cellulose), and mixtures thereof.

It is particularly preferred that the powdered hygroscopic material is a plant-based starch, which may be native, modified, or a mixture thereof. Desirable plant-based starches for use in the personal care compositions disclosed herein include *Maranta arundinacea* (Arrowroot) powder, *Manihot esculenta* (Cassava) starch, *Zea mays* (Corn) starch, *Oryza sativa* (Rice) starch, *Solanum tuberosum* (Potato) starch, *Triticum vulgare* (Wheat) starch, *Cycas revoluta* (Sago) starch, *Hordeum vulgare* (Barley) starch, or mixtures thereof. With respect to modified starches, physical modifications may include, in an illustrative but nonlimiting manner, annealing, freeze-thaw treatment, heat treatment (e.g., extrusion heating, simple oven heating, superheating), heat moisture treatment, hydrothermal treatment, mechanical treatment (e.g., grinding, mechanical activation, micronization, milling), moisture treatment, pH treatment, pressure treatment (e.g., dynamic pulsed pressure treatment, high pressure treatment, instantaneous controlled pressure treatment, osmotic pressure treatment, hydrostatic pressure treatment), pulsed-electric field treatment, radiation treatment (e.g., electromagnetic irradiation, gamma irradiation, microwave irradiation, UV irradiation), sonication and ultrasonic treatment, or a combination of different physical factors (e.g., duel modification). Desirable chemical modifications to such plant-based starches include, for example, techniques known by one of ordinary skill in the art to result in cationization, cross-linking, esterification (e.g., acetylation, fatty acetylation, phosphorylation, succinylation), etherification (e.g., methylation, hydroxymethylation, hydroxypropylation, carboxymethylation), graft copolymerization, hydrolysis, oxidation, or dual modification. Representative of the chemically modified starches are sodium hydroxypropyl starch phosphate and aluminum starch octenylsuccinate. Genetic variants of desirable plant-based starch, such as high amylose and high amylopectin starch, are also in scope for use in the disclosed composition. It is preferred that the chosen starch comprises arrowroot powder, cassava starch, corn starch, rice starch, potato starch, wheat starch, sodium hydroxypropyl starch phosphate, aluminum starch octenylsuccinate, or mixtures thereof. Preferably, the plant-based powder comprises arrowroot powder. It is particularly preferred that the plant-based starch is arrowroot powder.

Desirable cellulosics include hydroxypropyl cellulose, hydroxypropyl methylcellulose, ethylcellulose and sodium carboxy methylcellulose. Desirable sources of cellulose microfibrils include secondary cell wall materials (e.g., wood pulp, cotton), bacterial cellulose, and primary cell wall materials. Preferably, the source of primary cell wall material is selected from parenchymal tissue from fruits, roots, bulbs, tubers, seeds, leaves and combination thereof; more preferably is selected from citrus fruit, tomato fruit, peach fruit, pumpkin fruit, kiwi fruit, apple fruit, mango fruit, sugar beet, beet root, turnip, parsnip, maize, oat, wheat, peas and combinations thereof; and even more preferably is selected from citrus fruit, tomato fruit and combinations thereof. A most preferred source of primary cell wall material is parenchymal tissue from citrus fruit. Citrus fibers, such as those made available by Herbacel ^{®} as AQ Plus can also be used as source for cellulose microfibrils. The cellulose sources can be surface modified by any of the known methods including those described in Colloidal Polymer Science, Kalia et al., "Nanofibrillated cellulose: surface modification and potential applications" (2014), Vol 292, Pages 5-31.

In a preferred aspect of the disclosed personal care composition, the composition comprises a high total powder load. As used herein, powder load is used in the field of the art and refers to the ratio of all powder components of a composition to the total contents of the composition. It is preferred that the composition comprises at least 40% total powder components (e.g., the combination of the first mineral compound, the second mineral compound, inorganic salt, and powdered hygroscopic material) by weight of the composition, preferably, 45 to 60%, and, more preferably, 47 to 58% by weight of the personal care composition. In a preferred aspect, the total powder load of the composition ranges from 50 to 55% by weight of the personal care composition.

Personal care compositions as disclosed herein comprise at least one oil, wax, or mixture thereof. It is preferred that the oil or wax is plant-based. Illustrative but nonlimiting examples of plant-based oils and waxes include *Adansonia digitata* (Baobab) seed oil, alkanes, *Argania spinosa* (Argan) kernel oil, *Astrocaryum murumuru (Murumuru) butter, Astrocaryum aculeatum (Tucuma) butter,* beeswax, *Borago officinalis* (Borage) seed oil, *Brassica campestris* (Rapeseed) seed oil, *Butyrospermum parkii* (Shea) butter, *Calophyllum inophyllum* (Tamanu) seed oil, *Camelina sativa* (False Flax) seed oil, caprylic/capric triglyceride, *Carthamus tinctorius* (Safflower) seed oil, *Copernicia cerifera* (Carnauba) Wax, coco-caprylate/caprate, *Cocos nucifera* (Coconut) oil, *Crambe abyssinica* (Crambe) seed oil, *Euphorbia cerifera* (Candelilla) wax, *Euterpe oleracea* (Açaí) fruit oil, *Garcinia indica* (Kokum) seed butter, *Gossypium herbaceum* (Cotton) seed oil, *Helianthus annuus* (Sunflower) seed oil, *Helianthus annuus* seed wax, *Hippophae rhamnoides* (Sea Buckthorn) oil, jojoba esters, *Limnanthes Alba* (Meadowfoam) seed oil, *Linum usitatissimum* (Linseed) seed oil, *Macadamia temifolia* (Macadamia) seed oil, *Mangifera indica* (Mango) seed butter, *Moringa oleifera* (Moringa) seed oil, *Myrica cerifera* (Bayberry) fruit wax, *Oenothera biennis* (Evening Primrose) oil, *Olea europaea* (Olive) fruit oil, *Orbignya oleifera* (Babassu) seed oil, *Oryza sativa* (Rice) bran wax, *Oryza sativa* (Rice) germ oil, *Persea gratissima* (Avocado) oil, *Platonia insignis* (Bacuri) seed butter, *Plukenetia volubilis* (Sacha inchi) seed oil, *Prunus amygdalus dulcis* (Sweet Almond) oil, *Prunus armeniaca* (Apricot) kernel oil, *Ricinus communis* (Castor) seed oil, *Rosa canina* (Rosehip) seed oil, *Rosa damascena* flower wax, *Rosa moschata* (Rosehip) seed oil, *Salvia hispanica* (Chia) seed oil, *Salvia officinalis* (Sage) oil, *Schinziophyton rautanenii* (Mongongo) kernel oil, *Sesamum indicum* (Sesame) seed oil, *Shorea Robusta* (Sal) seed oil, *Simmondsia chinensis* (Jojoba) seed oil, soy lecithin, squalane, *Theobroma* Cacao (Cocoa) butter, *Theobroma grandiflorum* (Capuagu) seed butter, Triolein (algae oil), *Triticum vulgare* (Wheat) germ oil, *Virola surinamensis* (Ucuuba) seed butter, *Vitis vinifera* (Grapeseed) oil, *Zea mays* (Corn) oil and mixtures thereof. Desirable oils or waxes for use in the disclosed personal care compositions also include those from synthetic origins, preferably, isopropyl myristate, isopropyl palmitate, silicones, alkanes, and mixtures thereof. Preferably, the oil or wax comprises shea butter. Preferably, the oil or wax comprises candelilla wax, caprylic/capric triglyceride, coconut oil, jojoba seed oil, shea butter, sunflower oil, or mixtures thereof. Preferably, the oil, wax or mixture thereof used in accordance with the disclosed personal care composition is characterized by a melting point of 35 to 60°C, and, more preferably, a melting point of 40 to 55°C. The oil, wax or mixtures thereof may be incorporated in the disclosed personal care composition in total amounts ranging from 30% to 65%, preferably, from 35 to 60%, and, more preferably, from 40% to 55%, oil, wax or mixtures thereof by weight of the composition. In another aspect, it is provided that the disclosed personal care composition comprises 40 to 50% oil, wax or mixtures thereof by weight of the composition.

Personal care compositions as disclosed herein are oil continuous. Preferably, the composition is anhydrous, being substantially free of water. As used herein, "substantially free of water" means compositions having less than 5% by weight water, for example from 0 wt% to 5 wt%, preferably, less than 1%, for example from 0 wt% to 1 wt%, more preferably, less than 0.5%, for example from 0 wt% to 0.5 wt% still more preferably, less than 0.1%, for example from 0 wt% to 0.1 wt% and, most preferably, 0% by weight of water.

In a preferred aspect, the disclosed personal composition is natural, naturally derived, or a combination thereof.

The personal care composition is free of astringent metal salts comprising aluminum and/or zirconium. The personal care composition does not comprise an astringent metal salt selected from the group consisting of aluminum chloride, aluminum hydrochloride, aluminum chlorohydrate, aluminum chlorohydrex polyethylene glycol, aluminum chlorohydrex propylene glycol, aluminum dichlorohydrate, aluminum dichlorohydrex polyethylene glycol, aluminum dichlorohydrex propylene glycol, aluminum sesquichlorohydrate (ASCH), aluminum sesquichlorohydrex polyethylene glycol, aluminum sesquichlorohydrex propylene glycol, aluminum zirconium octachlorohydrate. aluminum zirconium octachlorohydrex gly, aluminum zirconium pentachlorohydrate, aluminum zirconium pentachlorohydrex gly, aluminum zirconium tetrachlorohydrex gly, aluminum zirconium tetrachlorohydrate, aluminum zirconium trichlorohydrate, aluminum zirconium trichlorohydrex gly. Many of such actives are listed in the U.S. Food and Drug Administration (FDA) antiperspirant drug products for over-the-counter human use monograph under C.F.R. Title 21, Sec. 350.10. The disclosed personal care compositions also do not comprise ASCH comprising water-soluble calcium salt and amino acid, in particular glycine. Such salts are preferable of enhanced activity, achieved by heating the ASCH with the water- soluble calcium salt and amino acid at sufficient temperature and for sufficient time for the antiperspirancy performance of the ASCH to be improved. Further details on this technology may be found in WO 2014/187685 by Unilever.

Preferably, the disclosed personal compositions are substantially free of added alcohol (e.g., excluding alcohol residual from the manufacturing or extraction processes of a raw material or blend of raw materials). It is particularly preferred that the personal care compositions have less than 1%, more preferably, 0.1%, and, most preferably, 0% alcohol by weight of the composition. "Alcohol," as used herein, consists essentially of methyl alcohol (methanol), ethyl alcohol (ethanol), isopropyl alcohol, alcohol denatured or specifically denatured (SD) alcohol.

The disclosed personal care composition can comprise other optional components that is known for use in deodorant or antiperspirant compositions, or which is otherwise desirable for topical application to the skin. The CTFA Personal Care Ingredient Handbook, Second Edition, 1992, describes a wide variety of non-limiting personal care and pharmaceutical ingredients commonly used in the personal care industry. Illustrative examples include but are not limited to antioxidants, astringents, binders, biological additives, buffering agents, chelators, colorants/dyes, conditioners, emollients, essential oils, exfoliating agents, fragrances, humectants, natural extracts, pH adjusters, polymers, preservatives, sensory modifiers (e.g., silicones), skin appearance modifiers (e.g., skin-lightening agents and skin-smoothing agents), solvent, surfactant, suspending agent, viscosity modifiers, and vitamins. Such an optional component may be included from about 0.001% to 10%.

A preferred optional component in the disclosed personal care composition is a malodor control active, antioxidant, or mixture thereof, which may be used at 0.01 to 1%, preferably, 0.025 to 0.75%, more preferably, 0.05 to 0.3% by weight of the composition. Such a malodor control active desirable for use preferably includes TMM^{®} (Total Malodor Management) and All-Natural TMM^{®}, which are both blends comprising triethyl citrate, made commercially available by the supplier Belle Aire.

It is also within the scope of the disclosed personal care compositions to further include one or more antioxidants, which may be natural, naturally derived, or synthetic antioxidants. Antioxidants may be any antioxidant desirable for use in personal care compositions. Illustrative but nonlimiting examples of such antioxidants comprise alpha hydroxy acids (e.g., citric acid, lactic acid, glycolic acid and mandelic acid), amino acids and derivatives thereof, beta-hydroxy acids (e.g., propanoic acid and salicylic acid), butylated hydroxytoluene (BHT), carotenes, carotenoids, co-enzyme Q10, didodecyl 3,3' thiodipropionate, glutathione, imidazole and derivatives thereof, octadecyl di-t-butyl-4-hydroxyhydrocinnamate, pentaerythrityl tetra-di-t-butyl hydroxyhydrocinnamate, peptides and derivatives thereof, plant extracts (e.g., *Rosmarinus officinalis* (rosemary) leaf extract and *Camellia sinesis* (green tea) leaf extract), polyhydroxy acids (e.g., gluconic acid, gluconolactone and lactobionic acid), polyphenols (e.g., anthocyanin, ellagic acid, flavonoid, tannin), and resveratrol.

Another class that may be used in the personal care composition as antioxidants includes vitamins. Illustrative vitamins include, but are not limited to, vitamin B₂, vitamin B₅ (panthenol), vitamin B₆, vitamin C, vitamin E, vitamin F, vitamin K, folic acid and biotin. Derivatives of the vitamins may also be employed. For instance, vitamin C derivatives include ascorbyl tetraisopalmitate, magnesium ascorbyl phosphate and ascorbyl glycoside. Derivatives of vitamin E include tocopheryl acetate, tocopheryl palmitate and tocopheryl linoleate. It is preferred that the antioxidant comprises vitamin E and derivatives thereof.

Fragrance is also a desirable optional component. The term "fragrance" is defined herein as odoriferous compounds or a mixture of odoriferous compounds, optionally mixed with a desirable solvent diluent or carrier, which is employed to impart a desired odor. A perfumery molecule or a group of perfumery molecules formulated as a perfume is generally incorporated in most personal care compositions. Any perfumery molecule / fragrance which is cosmetically acceptable may be included in the disclosed personal care composition disclosed herein. Desirable materials include conventional perfumes, such as perfume oils and also include so-called deo-perfumes, as described in EP 545,556 and other publications. Highly preferred fragrance components used in personal care compositions are cyclic and acyclic terpenes and terpenoids. These materials are based upon isoprene repeating units. Examples include alpha and beta pinene, myrcene, geranyl alcohol and acetate, camphene, dl-limonene, alpha and beta phellandrene, tricyclene, terpinolene, allocimmane, geraniol, nerol, linanool, dihydrolinanool, citral, ionone, methyl ionone, citronellol, citronellal, alpha terpineol, beta terpineol, alpha fenchol, borneol, isoborneol, camphor, terpinen- 1-ol, terpin-4-ol, dihydroterpineol, methyl chavicol, anethole, 1,4- and 1,8-cineole, geranyl nitrile, isobornyl acetate, linalyl acetate, caryophyllene, alpha cedrene, guaiol, patchouli alcohol, alpha and beta santalol, and mixtures thereof. Levels of incorporation are preferably up to 4%, particularly from 0.1% to 3%, and especially from 0.7% to 2%.

Other additional optional skin benefit agents desirable for use in the disclosed personal care composition include minerals and skin nutrients such as magnesium, calcium, copper, zinc and other metallic components; retinoic acid and its derivatives (e.g., cis and trans); retinal; retinol; retinyl esters such as retinyl acetate, retinyl palmitate, and retinyl propionate; resorcinol derivatives (particularly 4-substituted resorcinol derivatives, including 4-ethyl resorcinol, 4-isopropyl resorcinol, 4-hexyl resorcinol, 4-cyclopentyl resorcinol, thiamidol, 4-cyclohexyl resorcinol and acylated forms thereof); resveratrol, saccharide isomerate, ceramides (e.g. Ceramide 1, Ceramide 3, Ceramide 3B and Ceramide 6) and pseudoceramides, allantoin, pyroglutamic acid (PCA) salt derivatives including zinc PCA and sodium PCA, 12-hydroxystearic acid, petroselinic acid, conjugated linoleic acid, octadecanoic acid, hyaluronic acid and its salt derivatives mixtures thereof and the like. Such skin benefit agents, when used, collectively make up from 0.001 to 12% by weight of the personal care composition.

A wide selection of botanical extracts may optionally be included in the personal care compositions disclosed. The extracts may either be soluble in water or oil, carried in a solvent that is hydrophilic or hydrophobic, respectively, provided that they are compatible with a personal care composition that is oil-continuous or anhydrous. In the preferred aspect, water or ethanol are the extract solvents. Illustrative examples include those extracted from green tea, yarrow, chamomile, licorice, aloe vera, citrus unshui, willow bark, hops, alfalfa, algae, witch hazel, sage, thyme, and rosemary, as well as oils such as those derived from sea buckthorn, moringa, argan, avocado, calendula, algal, eucalyptus, lavender, bergamot, and marula. Soy extracts may be used and especially when it is desirable to include retinol. Such extracts, when used, are preferably employed in collective amounts ranging from 0.001 to 12% by weight of the personal care composition.

Another optional component desirable for use includes hemp oil with 2.5 to 25% by weight cannabigerol and/or cannabidiol at from 0.5 to 10 percent by weight. When used, such oil makes up from 0.0001 to 12% by weight of the composition, and preferably, from 0.01 to 5% by weight of the personal care composition.

Personal care chemists are familiar with appropriate preservatives and routinely choose them to satisfy preservative tests and product stability tests. Preservative systems should be selected having regard for the use of the composition and possible incompatibilities between the preservatives and other ingredients in the formulation. Exemplary examples of preservatives for use in anhydrous compositions include, without limitations, butyl paraben, ethyl paraben, methyl paraben, and propyl paraben. Preservatives are preferably employed in amounts ranging from 0.01 to 2% by weight of the personal care composition. It is particularly preferred that the personal care composition is substantially free of preservatives, or, more preferably, is preservative-free.

In another aspect of the personal care compositions disclosed herein, there is provided a method of attaining deodorizing benefits comprising the steps of: (a) identifying skin in need of deodorization, preferably, the axillae; and (b) topically applying, directly or indirectly, to the skin a composition made according to the first aspect of the compositions disclosed herein.

In yet another aspect, there is provided a use of the disclosed personal care composition for delivering a deodorancy benefit.

In still another aspect, there is provided a method of manufacturing the disclosed personal care composition.

In addition, it has also been found that the disclosed personal care compositions may provide an improved sensory feel at application and throughout the day. "Improved sensory feel," as used herein, refers to a reduced sticky, tacky, and/or greasy sensorial experience upon topical application of the composition and during the entire duration of use.

The stability of the personal care compositions disclosed herein were assessed for changes in color, odor, and appearance of the composition at 45°C for 12 weeks. Samples were placed in ovens at desirable temperatures for 12 weeks and allowed to cool to room temperature (e.g., approximately 25°C) and settle for 24 hours before assessing stability. Resulting samples were assessed with a 25°C control. Physical appearance is a key attribute to pass: samples must have a smooth surface and have no visible cracks in the composition or separation of the oil and wax structure to pass.

Personal care compositions disclosed herein possess a density ranging from 0.8 to 1.7 kg/L, preferably 1.0 to 1.6 kg/L. It is particularly preferred that the density ranges from 1.1 to 1.5 kg/L and, most preferably, from 1.15 to 1.4 kg/L.

A personal care composition as disclosed herein comprises:
(a) an inorganic salt;
(b) a first mineral compound, wherein the first mineral compound comprises a clay mineral;
(c) a second mineral compound, wherein the second mineral compound comprises a trioctahedral mineral;
(d) a powdered hygroscopic material; and
(e) a plant-based oil, wax, or mixtures thereof,

wherein the composition is substantially free of water, and
wherein a weight ratio of the first mineral compound to second mineral compound is 1:3 to 1:2.

In one aspect, a personal care composition as disclosed herein comprises:
(a) an inorganic salt;
(b) a first mineral compound, wherein the first mineral compound comprises a clay mineral;
(c) a second mineral compound, wherein the second mineral compound comprises a trioctahedral mineral;
(d) a powdered hygroscopic material; and
(e) a plant-based oil, wax, or mixtures thereof,

wherein the composition is substantially free of water, and
wherein a weight ratio of the first mineral compound to second mineral compound is 1:3 to 1:2.5.

In another aspect, a personal care composition as disclosed herein comprises:
(a) an inorganic salt, wherein the inorganic salt comprises an alkali or alkaline earth metal carbonate, or mixtures thereof;
(b) a first mineral compound, wherein the first mineral compound comprises a clay mineral;
(c) a second mineral compound, wherein the second mineral compound comprises a trioctahedral mineral;
(d) a powdered hygroscopic material; and
(e) a plant-based oil, wax, or mixtures thereof,

wherein the composition is substantially free of water,
wherein a weight ratio of the first mineral compound to second mineral compound is 1:3, and further wherein the weight ratio of powdered hygroscopic material to the plant-based oil or wax is 1:0.6 to 1:1.

Many types of packaging can be used to store and deliver the disclosed personal care composition. The selection of packaging is dependent upon the personal care end-use and the physical properties of the composition itself. The personal care product is in the form of a stick; preferably, comprising the composition as hereinbefore described comprised within a stick dispenser product package. Usual stick products are packaged so that there is a plastic dome on top of the stick, between the solid stick formulation and the cap. The dome is removed and typically discarded by the consumer prior to first use. There is also provided a cap which can be replaced following each use.

In an embodiment of the invention the personal care composition is for a deodorant product. In a preferred embodiment the personal care composition is for a personal care stick product. In a more preferred embodiment, the personal care composition is for a deodorant stick product.

In a preferred embodiment of the invention the personal care composition comprises:
(a) an inorganic salt;
(b) a first mineral compound, wherein the first mineral compound comprises a clay mineral;
(c) a second mineral compound, wherein the second mineral compound comprises a trioctahedral mineral;
(d) a powdered hygroscopic material; and
(e) a plant-based oil, wax, or mixtures thereof,

wherein the composition is substantially free of water, and
wherein a weight ratio of the first mineral compound to second mineral compound is 1:3 to 1:2, preferably, 1:3 to 1:2.5.

Wherein the inorganic salt is an alkali or alkaline earth metal carbonate, preferably, magnesium carbonate, calcium carbonate, sodium carbonate, sodium bicarbonate, or a mixture thereof in amounts of 0.25 to 15%, preferably, 0.5 to 12%, more preferably, 1 to 9%, and, most preferably, 1.5 to 6% by weight of the composition.

Wherein the composition comprises from 0.01 to 25% of the first mineral compound, preferably, 0.1 to 20%, and, most preferably, 0.5 to 15% first mineral compound by weight of the personal care composition.

Wherein the clay mineral is a crystalline silicate, preferably, an asymmetric 1:1 or 2:1:1 clay particle comprising alternating tetrahedral and octahedral sheets terminating with a tetrahedral sheet at one exterior surface plane and an octahedral sheet at another exterior surface plane.

Wherein the first mineral compound is kaolin, bentonite, or a mixture thereof.

Wherein the second mineral compound is a trioctahedral mineral having an octahedra containing a divalent cation.

Wherein the second mineral compound is an oxide mineral, preferably, magnesium hydroxide and/or calcium hydroxide, and is present in amounts from 1 to 50%, preferably, 2 to 40%, more preferably, 3 to 35% of the second mineral compound by weight of the composition.

Wherein the first and second mineral compounds have average particle sizes ranging from 0.001 to 60 microns, preferably, 0.01 to 50 microns.

Wherein the powdered hygroscopic material is arrowroot powder, cassava starch, corn starch, rice starch, potato starch, wheat starch, or mixtures thereof, preferably, arrowroot powder, in amounts ranging from 0.5 to 40%, preferably, from 1 to 38%, more preferably, from 2 to 37% by weight of the personal care composition.

Wherein the composition comprises a total powder load of at least 40%, preferably, 45 to 60% total powder components by weight of the personal care composition.

Wherein the plant-based oil, wax, or mixture thereof comprises candelilla wax, caprylic/capric triglyceride, coconut oil, jojoba seed oil, shea butter, sunflower oil, or mixtures thereof, and is present in amounts from 30% to 65%, preferably, from 35 to 60%, and, more preferably, from 40% to 55%, plant-based oil or wax by weight of the composition.

Wherein the personal care composition has a weight ratio of the powdered hygroscopic material to the plant-based oil or wax of 1:0.6 to 1:1, preferably, 1:0.7 to 1:0.9.

Wherein the personal care composition does not comprise an astringent metal salt comprising aluminum, zirconium, or a mixture thereof.

Wherein the personal care composition has a density ranging from 0.8 to 1.7 kg/L, preferably, 1.0 to 1.6 kg/L, more preferably, 1.1 to 1.5 kg/L.

Wherein the personal care composition is a deodorant stick that is natural, naturally derived, or a combination thereof.

In an embodiment of the invention the personal care composition comprises:
(a) from 0.01 wt% to 15 wt% magnesium carbonate, sodium bicarbonate or magnesium carbonate and sodium bicarbonate;
(b) from 0.01 wt% to 25 wt% kaolin;
(c) from 1 wt% to 50 wt% magnesium hydroxide;
(d) from 0.5 wt% to 42 wt% arrowroot and
(e) from 30 wt% to 65 wt% shea butter, caprylic/capric triglyceride, coconut oil, candelilla wax, jojoba seed oil and sunflower oil,
(f) from 0 wt% to less than 5 wt% water, and
wherein a weight ratio of kaolin to magnesium hydroxide is from 1:3 to 1:2, preferably, from 1:3 to 1:2.5 and the magnesium hydroxide is not a component of a clay mineral.

### Examples

### Example 1. Sample formulations in scope of the disclosed personal care compositions

| Ingredient | Weight percent (wt%) | | | | | | |
|---|---|---|---|---|---|---|---|
| | Sample | | | | | | |
| | 1 | 2 | 3 | 4 | 5 | 6 | 7 |
| *Butyrospermum parkii* (Shea) butter | 5.50 | 5.50 | 5.50 | 5.50 | 5.50 | 5.50 | 5.50 |
| Plant-based oil/wax blend (caprylic/capric triglyceride, coconut oil, candelilla wax, jojoba seed oil, and sunflower oil) | Balance | | | | | | |
| Magnesium hydroxide | 34.0 | 8.50 | 17.0 | 28.2 | 8.50 | 8.50 | 17.0 |
| Arrowroot powder | 6.00 | 36.8 | 28.6 | 11.8 | 40.0 | 41.8 | 24.9 |
| Kaolin | 11.3 | 2.82 | 5.64 | 11.3 | 2.82 | 2.82 | 5.64 |
| Magnesium carbonate | 2.50 | 0.63 | 2.50 | 2.50 | 2.50 | 0.63 | 1.25 |
| Sodium bicarbonate | - | - | - | - | - | - | 5.00 |
| Malodor control active + antioxidant | 1.30 | 1.30 | 1.30 | 1.30 | 1.30 | 1.30 | 1.30 |
| | | | | | | | |
| Clay mineral to trioctahedral mineral ratio | 1:3 | 1:3 | 1:3 | 1:2.5 | 1:3 | 1:3 | 1:3 |
| Stable @ 45°C for 12 weeks | Yes | Yes | Yes | Yes | Yes | Yes | Yes |

Personal care compositions were made by combining the above ingredients in accordance with the present personal care compositions. The inclusion levels and specific identities of the first mineral compound; the second mineral compound; powdered hygroscopic material; and plant-based oil, wax, mixture thereof may be varied to observe the impact of each ingredient on the overall stability of the composition at 45°C for 12 weeks based on assessments of color, odor, and appearance.

### Example 2. Sample formulations not in scope of the disclosed personal care compositions

| Ingredient | Weight percent (wt%) | | | |
|---|---|---|---|---|
| | Sample | | | |
| | A | B | C | D |
| *Butyrospermum parkii* (Shea) butter | 5.50 | 5.50 | 5.50 | 5.50 |
| Plant-based oil/wax blend (caprylic/capric triglyceride, coconut oil, candelilla wax, jojoba seed oil, and sunflower oil) | Balance | | | |
| Magnesium hydroxide | 36.5 | 34.0 | 39.5 | 8.50 |
| Arrowroot powder | 6.00 | 8.50 | 0.55 | 42.5 |
| Kaolin | 11.3 | 11.3 | 11.3 | 2.82 |
| Magnesium carbonate | - | - | 2.50 | - |
| Malodor control active + antioxidant | 1.30 | 1.30 | 1.30 | 1.30 |
| | | | | |
| Clay mineral to trioctahedral mineral ratio | 1:3.2 | 1:3 | 1:3.5 | 1:3 |
| Stable @ 45°C for 12 weeks | No | No | No | No |

Deodorant compositions were made by combining the above ingredients in accordance with the disclosed personal care compositions. The overall stability of the composition at 45°C for 12 weeks. Samples A, B, and D do not include inorganic salt such as magnesium carbonate, resulting in instable compositions out of scope and in scope of the disclosed ratio of clay mineral to trioctahedral mineral. For Sample D, the commercially available magnesium hydroxide used has an average particle size exceeding 60 microns. The clay mineral to trioctahedral mineral ratio in Sample C is out of scope according to the personal care composition disclosed herein despite the presence of magnesium carbonate.

### Example 3. Sensory testing

A series of clinical experiments were conducted to evaluate personal care compositions as disclosed herein against those out-of-scope of the disclosed personal care compositions. The clinical involved trained panelists. Panelists apply a single dose of 0.3 mL of the sample to be evaluated on an applicator to gentle spread the product on a designated underarm. Assessments may then be made at various time points during and after application. Perceived drying time was then evaluated by the panelists.

| Sample | Perceived drying time - least sq. mean (second) | Lower 95% | Upper 95% |
|---|---|---|---|
| I. Secret ^{®} Derma+ Invisible Gel Antiperspirant and Deodorant | 323 | 317 | 329 |
| II. Tom's of Maine ^{®} Deodorant Fragrance-Free, 24-hour odor protection | 593 | 587 | 599 |
| III. Native^{™} Unscented Deodorant | 117 | 111 | 123 |
| Sample 1 unfragranced | 93 | 87 | 99 |

Comparison of a composition made according to the personal care compositions disclosed herein with various competitor product benchmark were made in terms of perceived drying time post-application to the axilla area. Competitor product I is a propylene glycol-based antiperspirant gel containing aluminum salts for antiperspirancy. Competitor product II is a conventional propylene glycol-based (i.e., water-based) deodorant stick without aluminum salts. Competitor product III is a natural oil-based deodorant product without aluminum salts. Results demonstrate that personal care compositions made in accordance with those disclosed herein produce superior perceived drying times when compared to such conventional formulations available commercially. A quicker perceived drying time correlates to an enhanced sensory experience upon application associated with increased perception of dryness.

## Claims

1. A personal care stick product comprising a personal care composition comprising:
(a) from 0.01 wt% to 15 wt% inorganic salt,
(b) from 0.01 wt% to 25 wt% of a first mineral compound, wherein the first mineral compound comprises a clay mineral;
(c) from 1 wt% to 50 wt% of a second mineral compound, wherein the second mineral compound comprises an oxide mineral;
(d) from 0.5 wt% to 42 wt% powdered hygroscopic material selected from the group consisting of diatomaceous earth, silica, plant-based starch, non-modified cellulose and mixtures thereof;
(e) from 30 wt% to 65 wt% plant-based oil, wax, or mixtures thereof, and
(f) from 0 wt% to less than 5 wt% water,
wherein the weight ratio of the first mineral compound to second mineral compound is from 1:3 to 1:2 and the second mineral compound is not a clay mineral.

2. A personal care composition according to claim 1, wherein the inorganic salt is selected from an alkali metal salt, an alkaline earth metal salt and mixtures thereof.

3. A personal care composition according to claim 1 or 2, wherein the inorganic salt is selected from the group consisting of magnesium carbonate, calcium carbonate, sodium carbonate, sodium bicarbonate and mixtures thereof.

4. A personal care composition according to claims 1 to 3, wherein the first mineral compound is a clay mineral selected from the group consisting of kaolin, bentonite, serpentine and mixtures thereof.

5. A personal care composition according to claims 1 to 4 comprising from 0.5 wt% to 15 wt% first mineral compound.

6. A personal care composition according to claims 1 to 5, wherein the second mineral compound is an oxide mineral selected from the group consisting of magnesium hydroxide, calcium hydroxide and mixtures thereof.

7. A personal care composition according to claims 1 to 6 comprising from 3 wt% to 35 wt% second mineral compound.

8. A personal care composition according to claims 1 to 7, wherein the powdered hygroscopic material is a plant-based starch selected from the group consisting of arrowroot powder, cassava starch, corn starch, rice starch, potato starch, wheat starch, and mixtures thereof.

9. A personal care composition according to claims 1 to 8, wherein the plant-based oil, wax, or mixtures thereof has a melting point of from 35 °C to 60 °C.

10. A personal care stick product composition according to claims 1 to 9 comprising form 0 wt% to 2 wt% water.

11. A personal care composition according to claims 1 to 10 comprising from 35 wt% to 60 wt% plant-based oil, wax, or mixtures thereof.

12. A personal care composition according to claims 1 to 11, wherein the personal care composition does not comprise an astringent metal salt comprising aluminum, zirconium, or a mixture thereof.

13. A process for the preparation of a personal care composition according to claims 1 to 12.

14. Use of the personal care composition according to claims 1 to 12 for the reduction of malodor.

## Patentansprüche

1. Körperpflegeprodukt in Stiftform, das eine Körperpflegezusammensetzung umfasst, die
(a) 0,01 Gew.-% bis 15 Gew.-% anorganisches Salz,
(b) 0,01 Gew.-% bis 25 Gew.-% einer ersten mineralischen Verbindung, wobei die erste mineralische Verbindung ein Tonmineral umfasst;
(c) 1 bis 50 Gew.-% einer zweiten mineralischen Verbindung, wobei die zweite mineralische Verbindung ein oxidisches Mineral umfasst;
(d) 0,5 bis 42 Gew.-% pulverförmiges hygroskopisches Material, ausgewählt aus der Gruppe, bestehend aus Diatomeenerde, Siliciumdioxid, pflanzlicher Stärke, nicht modifizierter Cellulose und Mischungen davon;
(e) 30 Gew.-% bis 65 Gew.-% pflanzliches Öl, Wachs oder Mischungen davon und
(f) 0 Gew.-% bis weniger als 5 Gew.-% Wasser
umfasst,
wobei das Gewichtsverhältnis der ersten mineralischen Verbindung zur zweiten mineralischen Verbindung 1:3 bis 1:2 beträgt und die zweite mineralische Verbindung kein Tonmineral ist.

2. Körperpflegezusammensetzung nach Anspruch 1, wobei das anorganische Salz unter einem Alkalimetallsalz, einem Erdalkalimetallsalz und Mischungen davon ausgewählt ist.

3. Körperpflegezusammensetzung nach Anspruch 1 oder 2, wobei das anorganische Salz aus der Gruppe, die aus Magnesiumcarbonat, Calciumcarbonat, Natriumcarbonat, Natriumbicarbonat und Mischungen davon besteht, ausgewählt ist.

4. Körperpflegezusammensetzung nach den Ansprüchen 1 bis 3, wobei die erste mineralische Verbindung ein Tonmineral ist, das aus der Gruppe, die aus Kaolin, Bentonit, Serpentin und Mischungen davon besteht, ausgewählt ist.

5. Körperpflegezusammensetzung nach den Ansprüchen 1 bis 4, die 0,5 Gew.-% bis 15 Gew.-% erste mineralische Verbindung umfasst.

6. Körperpflegezusammensetzung nach den Ansprüchen 1 bis 5, wobei die zweite mineralische Verbindung ein oxidisches Mineral ist, das aus der Gruppe, die aus Magnesiumhydroxid, Calciumhydroxid und Mischungen davon besteht, ausgewählt ist.

7. Körperpflegezusammensetzung nach den Ansprüchen 1 bis 6, die 3 Gew.-% bis 35 Gew.-% zweite mineralische Verbindung umfasst.

8. Körperpflegezusammensetzung nach den Ansprüchen 1 bis 7, wobei das pulverförmige hygroskopische Material pflanzliche Stärke ist, die aus der Gruppe, bestehend aus Pfeilwurzelpulver, Maniokstärke, Maisstärke, Reisstärke, Kartoffelstärke, Weizenstärke und Mischungen davon, ausgewählt ist.

9. Körperpflegezusammensetzung nach den Ansprüchen 1 bis 8, wobei das pflanzliche Öl, Wachs oder Mischungen davon einen Schmelzpunkt von 35°C bis 60°C aufweisen.

10. Körperpflegezusammensetzung in Stiftform nach den Ansprüchen 1 bis 9, die 0 Gew.-% bis 2 Gew.-% Wasser umfasst.

11. Körperpflegezusammensetzung nach den Ansprüchen 1 bis 10, die 35 Gew.-% bis 60 Gew.-% pflanzliches Öl, Wachs oder Mischungen davon umfasst.

12. Körperpflegezusammensetzung nach den Ansprüchen 1 bis 11, wobei die Körperpflegezusammensetzung kein adstringierendes Metallsalz umfasst, das Aluminium, Zirkonium oder eine Mischung davon umfasst.

13. Verfahren zur Herstellung einer Körperpflegezusammensetzung nach den Ansprüchen 1 bis 12.

14. Verwendung der Körperpflegezusammensetzung nach den Ansprüchen 1 bis 12 zur Reduzierung üblen Geruchs.

## Revendications

1. Produit en stick de soins personnels comprenant une composition de soins personnels comprenant :
(a) de 0,01 % en poids à 15 % en poids d'un sel inorganique ;
(b) de 0,01 % en poids à 25 % en poids d'un premier composé minéral, le premier composé minéral comprenant un minéral argileux ;
(c) de 1 % en poids à 50 % en poids d'un deuxième composé minéral, ledit deuxième composé minéral comprenant un minéral oxyde ;
(d) de 0,5 % en poids à 42 % en poids d'un matériau hygroscopique en poudre choisi dans le groupe constitué par la terre de diatomée, la silice, l'amidon végétal, la cellulose non modifiée et des mélanges de ceux-ci ;
(e) de 30 % en poids à 65 % en poids d'une huile végétale, d'une cire ou de mélanges de celles-ci, et
(f) de 0 % en poids à moins de 5 % en poids d'eau,
dans lequel le rapport pondéral du premier composé minéral au deuxième composé minéral est de 1:3 à 1:2 et le deuxième composé minéral n'est pas un minéral argileux.

2. Composition de soins personnels selon la revendication 1, dans laquelle le sel inorganique est choisi parmi un sel de métal alcalin, un sel de métal alcalino-terreux et des mélanges de ceux-ci.

3. Composition de soins personnels selon la revendication 1 ou 2, dans laquelle le sel inorganique est choisi dans le groupe constitué par le carbonate de magnésium, le carbonate de calcium, le carbonate de sodium, le bicarbonate de sodium et des mélanges de ceux-ci.

4. Composition de soins personnels selon les revendications 1 à 3, dans laquelle le premier composé minéral est un minéral argileux choisi dans le groupe constitué par le kaolin, la bentonite, la serpentine et des mélanges de ceux-ci.

5. Composition de soins personnels selon les revendications 1 à 4 comprenant de 0,5 % en poids à 15 % en poids du premier composé minéral.

6. Composition de soins personnels selon les revendications 1 à 5, dans laquelle le deuxième composé minéral est un minéral oxyde choisi dans le groupe constitué par l'hydroxyde de magnésium, l'hydroxyde de calcium et des mélanges de ceux-ci.

7. Composition de soins personnels selon les revendications 1 à 6 comprenant de 3 % en poids à 35 % en poids du deuxième composé minéral.

8. Composition de soins personnels selon les revendications 1 à 7, dans laquelle le matériau hygroscopique en poudre est un amidon végétal choisi dans le groupe constitué par la poudre de maranta, l'amidon de manioc, l'amidon de maïs, l'amidon de riz, l'amidon de pomme de terre, l'amidon de blé et des mélanges de ceux-ci.

9. Composition de soins personnels selon les revendications 1 à 8, dans laquelle l'huile végétale, la cire végétale ou des mélanges de celles-ci ont un point de fusion de 35 °C à 60 °C.

10. Composition de produit en stick de soins personnels selon les revendications 1 à 9 comprenant de 0 % en poids à 2 % en poids d'eau.

11. Composition de soins personnels selon les revendications 1 à 10 comprenant de 35 % en poids à 60 % en poids d'une huile végétale, d'une cire ou de mélanges de celles-ci.

12. Composition de soins personnels selon les revendications 1 à 11, dans laquelle la composition de soins personnels ne comprend pas de sel métallique astringent comprenant de l'aluminium, du zirconium ou un mélange de ceux-ci.

13. Procédé de préparation d'une composition de soins personnels selon les revendications 1 à 12.

14. Utilisation de la composition de soins personnels selon les revendications 1 à 12 pour réduire les mauvaises odeurs.
